(19) Europäisches Patentamt
European Patent Office
Office européen des brevets

(11) Numéro de publication: **0 039 272**
**B1**

(12) # FASCICULE DE BREVET EUROPEEN

(45) Date de publication du fascicule du brevet: **18.04.84**

(51) Int. Cl.[3]: **C 07 C 87/38**, C 07 C 59/245, C 07 F 15/00, A 61 K 31/28

(21) Numéro de dépôt: **81400602.9**

(22) Date de dépôt: **15.04.81**

(54) **Composé complexe isocitraté du platine, son procédé de préparation et son application thérapeutique.**

(30) Priorité: **29.04.80 GB 8014109**

(43) Date de publication de la demande:
**04.11.81 Bulletin 81/44**

(45) Mention de la délivrance du brevet:
**18.04.84 Bulletin 84/16**

(84) Etats contractants désignés:
**DE NL SE**

(56) Documents cités:
**EP - A - 0 001 126**
**WO - A - 81/00256**
**FR - A - 2 401 933**
**US - A - 4 115 418**
**US - A - 4 169 846**

(73) Titulaire: **SANOFI, Société dite:, 40, Avenue George V, F-75008 Paris (FR)**

(72) Inventeur: **Macquet, Jean-Pierre, 4, Allée de la Soulane, Auzeville F-31320 Castanet-Tolosan (FR)**

(74) Mandataire: **Bressand, Georges et al, c/o CABINET LAVOIX 2 Place d'Estienne d'Orves, F-75441 Paris Cedex 09 (FR)**

**EP 0 039 272 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Composé complexe isocitraté du platine, son procédé de préparation et son application thérapeutique

La présente invention concerne un nouveau composé à base de platine, son procédé de préparation et son application thérapeutique dans le traitement des tumeurs malignes.

L'inhibition de la division cellulaire et l'activité antitumorale des complexes de coordination du platine, en particulier du cisdichlorodiamine-platine (II) ou PDD, de formule:

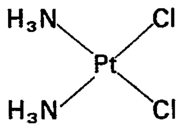

(III)

furent découvertes il y a une quinzaine d'années. Depuis lors, de nombreuses recherches ont porté sur cette nouvelle classe de composés en espérant:

1) identifier les plus actifs d'entre eux,

2) réduire les inconvénients qui ont été découverts rapidement (en particulier leur faible hydrosolubilité qui rend l'administration difficile, la néphrotoxicité et l'ototoxicité), et

3) élucider leur mécanisme d'action.

Des études récentes ont proposé divers changements dans la molécule. Par exemple, le remplacement des deux atomes de chlore du PDD par des ligands bidentates, tels que les groupements oxalato et malonato, a été proposé en 1973. En 1974, il a été suggéré de remplacer les deux molécules de $NH_3$ du PDD par le 1,2-diaminocyclohexane (DAC), par exemple dans le cisdichloro-(1,2-diaminocyclohexane)platine (II), dénommé ci-après PHD. Le complexe obtenu présentait l'avantage de ne pas offrir de résistance croisée avec le PDD chez l'animal. Deux autres complexes de platine, à savoir le cissulfatomonoaquo-(1,2-diaminocyclohexane)platine (II), dénommé ci-après PHS, et le cismalonato-(1,2-diaminocyclohexane)platine (II), dénommé ci-après PHM, sont également déjà connus.

Cependant, ces recherches n'ont pas été poursuivies à cause de la faible hydrosolubilité de ces composés et/ou de leur toxicité, en particulier leur toxicité rénale.

Des recherches conduites par l'auteur de la présente invention se sont orientées vers des ligands possédant un troisième groupement carboxylate afin d'améliorer l'hydrosolubilité du complexe. Ces ligands sont le citrate de sodium et le transaconitate de sodium. L'auteur du présent brevet a aussi synthétisé les différents complexes correspondant à la fois au cisdiaminoplatine (II) et au cis-(1,2-diaminocyclohexane)platine (II), en particulier le ciscitratodiamineplatine (II), dénommé ci-après PAC, le ciscitrato-(1,2-diaminocyclohexane)platine (II), dénommé ci-après PHC, et le cis(t-aconitato)-(1,2-diaminocyclohexane)-platine (II), dénommé ci-après PHTA. Il a pu notamment observer que le complexe citraté possédait une hydrosolubilité supérieure au PDD, mais que ces composés ne sont pas satisfaisants tant en ce qui concerne leur toxicité que leurs propriétés thérapeutiques, ainsi que cela sera mis en évidence ci-après par rapport au composé selon la présente invention.

L'auteur de la présente invention a également préparé les complexes obtenus à l'aide du ligand isocitrato, à savoir le cisisocitratodiamineplatine (II), dénommé ci-après PAIC, et le cisisocitrato-(1,2-diaminocyclohexane)platine (II), dénommé ci-après PHIC.

La présente invention repose sur la découverte surprenante que le complexe obtenu à partir du ligand isocitrate de sodium pour l'entité cis-(1,2-diaminocyclohexane)platine (II) présente à la fois une toxicité réduite et des propriétés antitumorales améliorées.

Le nouveau composé selon l'invention est le cis-isocitrato-(1,2-diaminocyclohexane)platine (II). Ce composé résulte de l'interaction entre une entité moléculaire dans laquelle l'atome de platine est déjà partiellement chélaté avec le 1,2-diaminocyclohexane (isomères trans-d, trans-l et trans-dl), représenté ci-dessous:

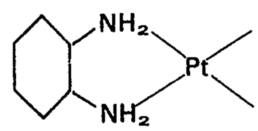

(I)

et l'isocitrate trisodique (isomères d; l; dl) correspondant à la formule:

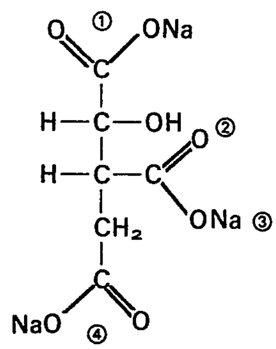

(II)

qui agit comme ligand labile. Deux liaisons peuvent se former entre l'entité moléculaire (I) et chacune des positions réactives 1, 2, 3 et 4 du ligand (II). La chélation a certainement lieu entre les positions 1 et 2 et les positions 1 et 3. Une interaction entre les positions 1-4, 3-4, 2-3 et 2-4 est aussi possible. Une fixation monodentate entre (I) et (II) est très probable: la première liaison étant une liaison Pt-carboxylate et la seconde liaison étant une liaison Pt-OH. Ainsi, l'invention se rapporte à chaque complexe possible, ainsi qu'au mélange de ces complexes en toutes proportions.

Le composé de l'invention, c'est-à-dire le PHIC, peut être préparé en solution aqueuse à partir du PHD de formule (IV) suivante:

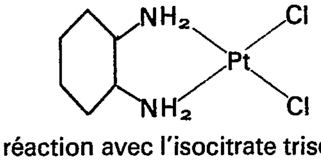

(IV)

par réaction avec l'isocitrate trisodique.

Selon une variante du procédé précédent, afin d'isoler sous forme solide le PHIC du milieu réactionnel, on opère d'après le schéma réactionnel suivant:

Dans une première étape, le dérivé diaquo est préparé d'après la réaction:

cis-Pt(DAC)$Cl_2$+2$AgNO_3$

→cis-[Pt(DAC)$(H_2O)_2$]$(NO_3)_2$+2AgCl

Dans une seconde étape, l'ion isocitrate est complexé suivant la réaction:

cis-[Pt(DAC)$(H_2O)_2$]$(NO_3)_2$+isocitrate

→PHIC·3$H_2O$+$NaNO_3$+$HNO_3$ trisodique 2$H_2O$

*Préparation du dérivé diaquo*

5 g de cis-Pt(DAC)$Cl_2$ sont mis en suspension dans 250 ml de $H_2O$ bidistillé, et on ajoute 4,3 g de $AgNO_3$ solide. La réaction s'effectue à l'abri de la lumière, pendant 4 h à 50-55°C. Il se forme un précipité blanc de AgCl. Le tout est mis à 0-4°C pendant quelques heures, puis filtré sur papier Whatman (Whatman est la marque commerciale de la compagnie Whatman Inc., New jersey, USA). Le précipité est lavé avec de l'eau (50-75 ml) et le filtrat (300-350 ml) incolore est passé sur filtre Millipore (0,45 µ) (Millipore est la marque commerciale de la société Millipore, Massachusetts, USA).

*Préparation du PHIC*

On ajoute 3,85 g d'isocitrate trisodique, 2$H_2O$, et on porte le mélange à 60-65°C pendant 20 h à l'abri de la lumière. La solution colorée en jaune (qui sera filtrée sur papier Whatman si nécessaire) est alors concentrée au rotavapor (température du bain = 50°C) jusqu'à 25-30 ml.

Le complexe est filtré sur Millipore (0,45 µ) et ajouté à l'aide d'une pipette (en agitant continuellement) à un mélange froid éthanol/acétone, 1/1, 1000 ml. L'ensemble est laissé à 4°C pendant 2 h, puis filtré sur fritté, lavé à l'acétone (100 ml), puis à l'éther (100 ml).

Le complexe de couleur blanc cassé est séché sous vide, puis pesé. On recueille environ 6,5 g de PHIC à partir de 5 g de cis-Pt(DAC)$Cl_2$, correspondant à un rendement de 85%.

Le PHIC ainsi obtenu sous forme de poudre blanc cassé commence à noircir lorsqu'on le chauffe à 245°C et se décompose complètement à 254°C. Les analyses élémentaires du PHIC sont reportées ci-dessous (le platine a été dosé par spectrophotométrie d'absorption atomique).

*Analyse* pour PHIC · 3$H_2O$:

Calculé:
C 24,26 H 4,10 N 4,72 Pt 32,84 Na 7,16%

Trouvé:
C 24,10 H 3,96 N 4,76 Pt 32,10 Na 6,99%

P.M. = 594,10

Ces résultats analytiques sont aussi en accord avec la formule PHIC · 2$H_2O$ dans laquelle l'entité Pt(DAC) ne forme pas deux liaisons avec l'ion isocitrate mais serait liée à un groupement carboxylate, certainement celui en position 1, l'autre liaison étant une liaison Pt−OH.

Ce composé possède une solubilité presque infinie dans l'eau (supérieure à 1500 mg/ml). Dans une solution de chlorure de sodium 9‰, cette solubilité est environ de 700 mg/ml; dans ce milieu, le complexe évolue en 24 h pour donner un précipité jaune correspondant au dérivé chloré. On peut identifier le PHIC à l'aide de ses chromatogrammes et par l'analyse des spectres U.V., I.R. et $^{13}C$ représentés sur les figures annexées.

La fig. 1 illustre des chromatogrammes liquide haute pression du PHIC qui ont été obtenus dans les conditions suivantes:

| Références | Concentration du solvant | Volume injecté (µl) | Sensibilité | Eluant | |
|---|---|---|---|---|---|
| | | | | Débit (ml/min) | Pression (MPa) |
| A | 0,5 mg/ml $H_2O$ | 25 | 0,02 | $H_2O$ 1,5 | 10,5 |
| B* | 0,5 mg/ml $C_2H_5OH/H_2$ (4/6) | 15 | 0,04 | $C_2H_5OH/H_2O$(4/6) 2 | 2,1 |
| C* | 0,5 mg/ml 1% acide acétique | 50 | 0,01 | 1% acide acétique 4 | 2,1 |
| D* | 0,5 mg/ml 1% acide acétique | 50 | 0,01 | 1% acide acétique 2 | 2,1 |

* Colonne $C_{18}$ reverse radiale.

La fig. 2 représente le spectre U.V. du PHIC enregistré sur un spectrophotomètre Cary 14, à une concentration de 3 mg/100 ml d'eau. Le coefficient d'absorption moléculaire est de 9400 $l \cdot mol^{-1} \cdot cm^{-1}$.

La fig. 3 représente le spectre I.R. du PHIC enregistré sur un spectrophotomètre Perkin Elmer 577. Le composé solide a été dilué à 1% dans du bromure de potassium, puis comprimé sous forme de pastille. Les deux pics standard correspondant à $\nu 1 = 2850{,}7\ cm^{-1}$ et $\nu 2 = 906{,}7\ cm^{-1}$.

La fig. 4 est un spectre en $^{13}C$ du PHIC obtenu d'après les conditions expérimentales suivantes:

Les spectres de résonance de $^{13}C$, avec découplage des protons, ont été obtenus à l'aide d'un spectromètre Bruker WH90 travaillant à 22,62 MHz en mode pulsé et avec transformée de Fourrier. Le nombre de signaux de libre précession qui ont été accumulés dans un domaine de 6 kHz en utilisant 8 kmots de mémoire était de 17 000 pour le PHIC. Une impulsion voisine de 90° pendant 12 µs a été utilisée avec des temps de répétition de 3 s.

Les déplacements chimiques δCi sont exprimés en parts par million et sont rapportés au TMS. Le PHIC a été dissous dans $D_2O$ à la concentration de 1,1 g/ml. La solution a été filtrée sur filtre Millipore (0,45 µ) avant l'enregistrement du spectre. $C_6D_6$ a été utilisé comme étalon externe. Les déplacements chimiques sont donnés dans le tableau ci-dessous.

*Déplacements chimiques δ $^{13}C$ (ppm/TMS) du PHIC*

| N° du pic | δCi | Attribution | |
|---|---|---|---|
| | | ligand | Ci |
| | PHIC | | |
| 1 | 184,2 | | |
| 2 | 183,9 | | |
| 3 | 183,8 | | |
| 4 | 182,9 | | |
| 5 | 182,7 | | |
| 6 | 182,4 | | |
| 7 | 182,3 | ISO* | C-1,2,3 |
| 8 | 182,2 | | |
| 9 | 182,0 | | |
| 10 | 181,9 | | |
| 11 | 75,9 | ISO | C-4 |
| | 75,2 | | |
| 12 | 64,8 | DAC** | C-1,1' |
| | 64,0 | | |
| 13 | 51,1 | | |
| | 50,6 | ISO | C-5 |
| | 50,3 | | |
| | 48,9 | | |
| 14 | 39,6 | | |
| | 39,1 | ISO | C-6 |
| | 38,4 | | |
| | 36,4 | | |
| 15 | 34,3 | | |
| | 34,1 | DAC | C-2,2' |
| | 33,4 | | |
| | 32,8 | | |
| 16 | 26,3 | DAC | C-3,3' |

* ISO = isocitrate de formule:

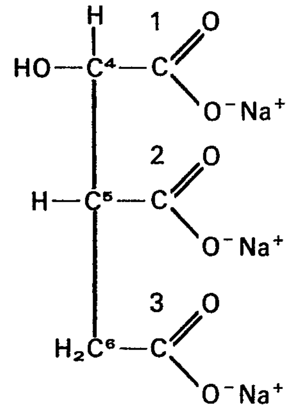

** DAC = 1,2-diaminocyclohexane de formule:

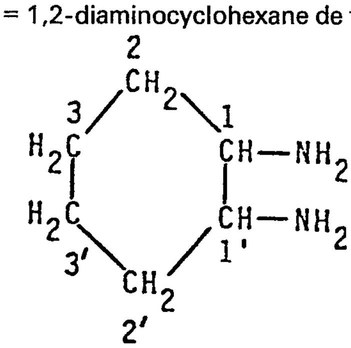

Les résultats des études pharmacologiques et toxicologiques rapportés ci-après mettent en évidence les propriétés du complexe PHIC selon l'invention. Ces études ont été menées sous forme comparative vis-à-vis de composés connus et mentionnés précédemment et ont permis de démontrer que le composé selon l'invention présente les avantages suivants:

1) une hydrosolubilité presque infinie qui est au moins plusieurs centaines de fois supérieure à celle d'autres complexes de platine similaires;

2) une très faible toxicité chez la souris et le babouin;

3) une activité antitumorale élevée envers la leucémie L1210 et le sarcome 180.

Ces études comparatives furent entreprises avec les composés qui sont identifiés par des abréviations à des fins de simplification de l'exposé.

*Etude pharmacologique*

Les propriétés antitumorales du PHIC ont été évaluées au cours d'études comparatives en utilisant une méthodologie classique sur deux types de tumeurs expérimentales chez la souris, la

leucémie L1210 et le sarcome 180. Les conditions dans lesquelles chaque expérimentation a été conduite ainsi que les résultats sont reportés ci-dessous.

I. *Leucémie L1210: Ascite et solide*

— Souris DBA/2 femelles (CNRS, Orléans) de 20 à 22 g,

— 10 souris par dose.

a) Greffe des cellules par voie i.p., traitement par voie i.p.*.

$10^5$ cellules sont greffées i.p. au jour $J_0$ et le traitement i.p. s'effectue 24 h après (jour $J_1$) en une seule injection.

b) Greffe des cellules par voie i.p., traitement par voie i.v.*.

$10^5$ cellules sont greffées i.p. au jour $J_0$ et le traitement i.v. (au niveau de la queue) s'effectue 24 h après (jour $J_1$) en une seule injection.

c) Greffe des cellules par voie s.c.*, traitement par voie i.p.

$10^6$ cellules sont greffées sous la peau derrière la tête de l'animal au jour $J_0$ et le traitement i.p. s'effectue en une seule injection, soit au jour $J_1$, soit au jour $J_4$.

II. *Sarcome 180: Ascite*

— Souris Swiss femelles (Evic-Ceba, Bordeaux) de 20 à 22 g,

— 10 souris par dose,

— $10^6$ cellules greffées i.p. au jour $J_0$,

— traitement i.p. 24 h après (jour $J_1$) en une seule injection.

Les composés de comparaison PDD, PAC, PAIC, PHC et PHTA, ainsi que le composé de l'invention PHIC, ont été dissous dans une solution de NaCl 9‰. PHD et PHM, qui sont également des composés de comparaison, ont été mis en suspension dans une solution de Klucel (hydroxypropylcellulose) à 4‰ (Klucel est une marque commerciale de la compagnie Hercules Inc., Delaware, USA). Le PHS a été dissous dans l'eau car sa dissolution dans le NaCl 4‰ entraîne la formation presque immédiate du composé chloré (très certainement monochloré, puis dichloré: PHD) jaune insoluble. Les solutions de ces composés ont été préparées extemporanément. Les augmentations du temps de vie des animaux traités (ILS) ont été calculées à partir du temps moyen de vie des animaux témoins en utilisant la formule:

$$ILS = \frac{\text{survie des animaux traités} - \text{survie des animaux témoins}}{\text{survie des animaux témoins}} \times 100$$

ILS = Increase Life Span = augmentation du temps de vie en pour-cent.

Les expériences ont été arrêtées 30 d après la greffe de la tumeur dans le cas de la leucémie L1210, et 60 d après la greffe de la tumeur dans le cas du sarcome 180. Les animaux survivants aux échéances précisées ci-dessus ont été inclus dans le calcul de l'ILS.

Les composés PAC, PAIC et PHTA ont donné 17% (100 mg/kg), 74% (75 mg/kg) et 13% (500 mg/kg) d'ILS sur la leucémie L1210 ($10^5$ cellules, greffe i.p. au $J_0$, traitement en une seule injection au $J_1$). A cause de leur inactivité (PAC et PHTA) ou de leur plus faible activité (PAIC) sur la L1210 que celle du PHIC, ces composés n'ont pas été étudiés plus en détail. Il est important de remarquer:

a) que le PHIC donne la meilleure activité antitumorale sur la leucémie L1210 et sur le sarcome S 180, greffe i.p., traitement i.p. (tableaux I et II);

b) que le PHIC est actif sur la leucémie L1210, lorsque la greffe est i.p. et le traitement i.v. (tableau III), et

c) que l'index thérapeutique du PHIC, défini par le rapport I.T. = $DL_{50}$/DME (DME = dose minimale efficace, c'est-à-dire la dose correspondant à 25% d'ILS pour les deux tumeurs expérimentales étudiées) est 5,5 fois supérieur à celui du PDD dans le cas de la leucémie L1210 et 4,0 fois supérieur à celui du PDD dans le cas du sarcome 180.

---

* i.p. = voie intrapéritonéale; i.v. = voie intraveineuse; s.c. = voie sous-cutanée.

*Tableau I*

Comparaison de l'activité antitumorale du PDD et du PHIC sur la leucémie L1210 et sur le sarcome 180

| | Dose (mg/kg) | L1210[a] | | S 180[b] | |
|---|---|---|---|---|---|
| | | ILS (%) | animaux survivants à 30 d (%) | ILS (%) | animaux survivants à 60 d (%) |
| PDD | 2 | 28 | 0 | — | — |
| | 4 | 52 | 0 | 41 | 0 |
| Comparaison | 6 | 75 | 0 | — | — |
| | 8 | 106 | 0 | 65 | 0 |
| PHIC | 5 | 16 | 0 | — | — |
| | 10 | 40 | 0 | 29 | 0 |
| Selon l'invention | 25 | 59 | 0 | 46 | 0 |
| | 50 | 79 | 0 | 112 | 20 |
| | 100[c] | 129 | 20 | 171 | 40 |

*Tableau I (suite)*

| |
|---|
| [a] Greffe i.p. ($10^5$ cellules) au Jour $J_0$ et traitement i.p. au jour $J_1$, 10 souris par dose.<br>Temps moyen de survie des animaux contrôlés: PDD = 9,3 ± 0,8 d; PHIC = 8,6 ± 0,4 d. |
| [b] Greffe i.p. ($10^6$ cellules) au jour $J_0$ et traitement i.p. au jour $J_1$, 10 souris par dose.<br>Temps moyen de survie des animaux contrôlés: PPD = 17,1 ± 2,0 d; PHIC = 18,7 ± 1,3 d. |
| [c] Une souris morte de toxicité, exclue du calcul de l'ILS. |

*Tableau II*

Comparaison de l'activité antitumorale de quelques dérivés Pt (DAC) sur la leucémie L1210 et sur le sarcome 180

| Composés de comparaison | Dose (mg/kg) | L1210 | | S 180 | |
|---|---|---|---|---|---|
| | | ILS (%) | animaux survivants à 30 d (%) | ILS (%) | animaux survivants à 60 d (%) |
| PDH | 1<br>2<br>4 | 45<br>49<br>65 | 0<br>0<br>0 | 100<br>128<br>127 | 0<br>20<br>10 |
| PHM | 5<br>10<br>20<br>40<br>50 | —<br>—<br>—<br>—<br>103 | —<br>—<br>—<br>—<br>10 | 25<br>36<br>44<br>60<br>95 | 0<br>0<br>0<br>0<br>0 |
| PHS | 0,5<br>1<br>2<br>4<br>5 | —<br>—<br>—<br>—<br>112 | —<br>—<br>—<br>—<br>40 | 4<br>10<br>25<br>47<br>112 | 0<br>0<br>0<br>0<br>10 |
| PHC | 10<br>25<br>50<br>100<br>120 | —<br>—<br>—<br>—<br>90* | —<br>—<br>—<br>—<br>0 | 20<br>124<br>121<br>—<br>— | 0<br>20<br>30<br>—<br>— |

* Sur 5 souris seulement.
Greffe i.p. ($10^5$ cellules pour la L1210 et $10^6$ cellules pour le S 180) au jour $J_0$ et traitement i.p. au jour $J_1$, 10 souris par dose.

*Tableau III*

Activité antitumorale du PHIC
sur la leucémie L1210
Greffe s.c. — Traitement i.p.
Greffe i.p. — Traitement i.v.

Greffe sous-cutanée ($10^6$ cellules)

| | ILS (%) |
|---|---|
| Traitement i.p. au $J_1$ | |
| 62,5 mg/kg | 26 |
| Traitement i.p. au $J_4$ | |
| 62,5 mg/kg | 17 |
| 125 mg/kg | 50 |

Temps moyen de survie des animaux contrôlés = 8,6 ± 0,5 d

Greffe intrapéritonéale ($10^5$ cellules)

| | ILS (%) |
|---|---|
| Traitement i.v. au $J_1$ | |
| 50 mg/kg | 25 |
| 125 mg/kg | 41 |

Temps moyen de survie des animaux contrôlés = 8,8 ± 0,7 d

*Etude de la toxicité*

Des études comparatives ont été entreprises afin d'évaluer les toxicités *in vitro* et *in vivo* du composé de la présente invention, le PHIC. Les résultats de ces études sont rapportés ci-dessous.

A — *Cellules L1210 en culture*

On apprécie la toxicité *in vitro* sur des cellules L1210 en culture en déterminant la dose inhibitrice 50 ($DI_{50}$) qui est la concentration du composé de platine qui réduit de 50% la vitesse de croissance des cellules après une exposition de longue durée à la drogue. Cette exposition est habituellement de 24 à 48 h. A la $DI_{50}$, toutes les cellules sont viables (exclusion au bleu de trypan). La partie expérimentale de ce travail est décrite dans un article récemment publié (P. Lecointe, J.P. Macquet et J.L. Butour (1979), «Biochem. Biophys. Res. Commun.», *90*, 209-213). Les résultats sont inclus dans le tableau IV.

*Tableau IV*

Cytotoxicité des différents composés sur les cellules L1210 en culture

| Composé de platine | | $DI_{50}$ (µm) |
|---|---|---|
| Comparaison | PDD | 2,33 |
| | PHD | 0,13 |
| | PHS | 0,19 |
| | PHM | 0,39 |
| Selon l'invention | PHIC | 0,42 |

B — *Toxicité chez la souris*

1. *Toxicité i.p., i.v. et v.o. **

L'étude toxicologique aiguë (une seule injection de composé) a été conduite sur des souris Swiss femelles (Evic-Ceba, Bordeaux) de 21 à 23 g et des rats. 10 animaux ont été traités pour chaque dose de composé. La toxicité des différents composés est donnée dans le tableau V.

* Voie orale.

*Tableau V*

Toxicité aiguë des différents composés chez la souris

| Composés | PDD | PAC | PAIC | PHD | PHC | PHIC | PHTA |
|---|---|---|---|---|---|---|---|
| $DL_0$* (mg/kg) | 9 | 125 | 75 | 7 | 150 | 135 ($DL_{10}$)** | >500 |

* $DL_0$ = dose maximale non mortelle, $DL_{10}$ = dose qui tue 10% des animaux.
** Voie orale.

Les toxicités intrapéritonéale (i.p.)[a], intraveineuse (i.v.)[a] et par voie orale (v.o.)[a] du PHIC sont reportées dans le tableau VI. La perte de poids au 4e d des animaux traités i.p. à la $DL_{10}$ est de 3,5 g et de 4,3 g à la $DL_{50}$.

[a] 10 animaux par dose.

(*Tableau VI en page suivante*)

2. *Toxicité rénale et hépatique*

La néphrotoxicité du PDD est bien connue. Il était alors intéressant de comparer les effets causés au niveau des reins et du foie, qui sont les organes dans lesquels le platine s'accumule le plus, par le composé de la présente invention et par les composés déjà connus cités précédemment.

On a utilisé des souris Swiss femelles (Evic-Ceba, Bordeaux) de 20 à 22 g. Les souris ont été traitées par voie intrapéritonéale au jour $J_0$, puis sacrifiées aux jours $J_1$, $J_4$ et $J_{10}$. 9 souris ont été utilisées par composé et les doses administrées sont reportées dans le tableau VII.

*Tableau VII*

Doses utilisées pour l'étude de la toxicité rénale et hépatique

| Composés | PDD | PAC | PHD | PHM | PHS | PHC | PHIC |
|---|---|---|---|---|---|---|---|
| Dose (mg/kg) | 8 | 100 | 4 | 50 | 5 | 120 | 150 |
| Solvant | solution aqueuse de NaCl 9‰ | | solution aqueuse de Klucel 4‰ | | $H_2O$ | $H_2O$ | solution aqueuse de Nacl 9‰ |

*Tableau VI*

Toxicités i.p., i.v. et v.o. du PHIC chez la souris et le rat

| Animal | Voie | $DL_{10}$ (mg/kg) | $DL_{50}$ (mg/kg) | $DL_{90}$ (mg/kg) |
|---|---|---|---|---|
| Souris | i.p. | 135 | 236 (220-254)* | 365 |
| | i.v. | 348 | 421 (411-431)* | 489 |
| | v.o. | | supérieure à 1000 | |
| Rat | i.p. | 61 | 143 (134-153)* | 278 |
| | i.v. | 111 | 173 (156-192)* | 244 |
| | v.o. | | supérieure à 1000 | |

* Limites de confiance pour P = 0,05.
$DL_{10}$ = dose qui tue 10% des animaux, $DL_{50}$ = dose qui tue 50% des animaux, $DL_{90}$ = dose qui tue 90% des animaux.

Il faut mentionner que ces doses correspondent à des doses maximales non toxiques chez la souris saine (voisines ou égales à la $DL_0$). Le contrôle des parenchymes rénaux et hépatiques fut réalisé sur des organes fixés au Duboscq-Brasil et inclus dans la paraffine. Les coupes de 5 µ d'épaisseur ont été colorées à l'hémalun éosine. En général, les modifications sont localisées au niveau du cortex et des tubes proximaux et distaux. Il s'agit:

a) *d'altérations nucléaires:*

— anisocaryose marquée,

— modification de la chromatine soit délavée, soit mottée avec hyperchromatisme et/ou pycnose,

— la membrane nucléaire est irrégulière et parfois incisée;

b) *d'altérations cytoplasmiques:*

— anisocytose avec modification de la bordure en brosse des tubes proximaux et inclusions éosinophiles intracytoplasmiques.

Quelques aspects rares suggèrent des nécroses cellulaires isolées dans les tubes proximaux et distaux. Un cas avec abcès a été observé sur un animal au jour $J_{10}$ avec le PHM. Des dilatations tubulaires, avec cylindres hyalins dans l'ensemble du néphron, sont retrouvées avec parfois un aspect pseudo-vésiculaire. Ces modifications, lorsqu'elles sont observées, culminent au jour $J_4$ et se stabilisent au $J_{10}$ avec des variations dans la même série suggérant des variations de sensibilité.

Ces altérations ont été observées avec:

a) PDD (8 mg/kg), PAC (100 mg/kg) et PHM (50 mg/kg);

b) PHD (4 mg/kg): modifications vasculaires sans cylindre hyalin, et

c) PHS (5 mg/kg): dilatation vasculaire et tubes contournés proximaux.

Le PHC (120 mg/kg) et le PHIC (150 mg/kg) n'ont pas, sur ce type de préparation, entraîné de modifications significatives. Les parenchymes spléniques et même surrénaliens, occasionnellement prélevés, n'ont pas permis de reconnaître d'anomalies.

Il ressort de cette étude que le nouveau composé de la présente invention, le PHIC, semble ne provoquer aucune lésion rénale, et ce à une dose 20 fois supérieure à celle du PDD. Des études comparatives de pénétration cellulaire *in vivo* chez la souris ont été réalisées avec le composé de la présente invention, le PHIC, ainsi qu'avec le PDD. Les résultats sont présentés ci-dessous.

Les souris (DBA/2) reçoivent $10^6$ cellules au jour $J_0$ et sont traitées 3 d après ($J_3$) par une quantité de composé de platine voisine de la $DL_0$ chez la souris saine. 2 h après le traitement au jour $J_3$, les animaux sont sacrifiés, les cellules sont extraites du péritoine de l'animal (environ 20 × $10^6$) et lavées plusieurs fois afin d'éliminer le platine extracellulaire, et aussi le platine intracellulaire qui ne serait pas lié de manière covalente avec les organites cellulaires. Le dosage du platine dans ces lavages s'effectue par absorption atomique. Le platine est ensuite dosé sur les cellules entières et sur le DNA après extraction au phénol. Dans le cas du PDD (9 mg/kg), il ne pénètre que 0,4% du platine injecté à l'animal, ce qui correspond à 180 × $10^{-16}$ g de platine par cellule leucémique. Dans ces conditions, on retrouve un atome de platine fixé pour 30 000 nucléotides, soit au total 1% de ce qui a pénétré est complexé à l'ADN. Dans le cas du PHIC (150 mg/kg), il pénètre 0,15% dans les cellules, ce qui correspond à 700 × $10^{-16}$ g de platine par cellule, et on retrouve un atome de platine fixé pour 3500 nucléotides. Comme dans le cas du PDD, 1% de ce qui a pénétré est complexé à l'ADN. Les résultats sont rassemblés dans le tableau VIII.

*Tableau VIII*

Comparaison de la pénétration du PDD et du PHIC dans les cellules L1210 greffées chez l'animal et quantification au niveau de l'ADN

| Composés de platine | Dose (mg/kg) | Quantité de platine pénétré par cellule (×$10^{16}$ g) | Nombre d'atomes de platine fixés par nucléotide sur l'ADN |
|---|---|---|---|
| PDD | 9 | 180 | 1/30000 |
| PHIC | 150 | 700 | 1/3500 |

C — *Toxicité chez le babouin*

Finalement, une étude toxicologique préliminaire a été conduite avec le PHIC chez le babouin afin de déterminer les paramètres suivants:

a) néphrotoxicité
b) hématotoxicité
c) toxicité gastro-intestinale
d) toxicité hépatique
e) ototoxicité

et aussi de définir la dose maximale tolérable (non mortelle).

Pour déterminer cette dose maximale tolérable, on a réalisé deux essais chez les babouins Nos 1 et 2, à des doses respectives de 100 et 150 mg/kg, les deux animaux n'ayant pas été préhydratés. Afin de vérifier l'effet bénéfique de l'hydratation, on a administré au babouin No 3 une dose de 200 mg/kg sous hydratation.

| Babouin No | Poids (kg) | Dose (mg/kg) | Fréquence des traitements |
|---|---|---|---|
| 1 | 8,4 | 100 | 6 { 1 au $J_0$<br>1 au $J_{37}$<br>1 au $J_{58}$<br>1 au $J_{84}$<br>1 au $J_{105}$<br>1 au $J_{133}$ |
| 2 | 7,3 | 150 | 1 au $J_0$ |
| 3 | 7,5 | 200 | 1 au $J_0$ |

Le PHIC a été dissous dans une solution de NaCl 9‰ à la concentration de 100 mg/ml, puis filtré sur filtre Millipore de 0,22 μ. Le composé est injecté par voie intraveineuse au niveau de la veine fémorale en 5 à 10 min. Le babouin No 3 (200 mg/kg) a reçu 700 ml de sérum physiologique par voie intraveineuse.

La toxicité gastro-intestinale est le premier type de toxicité qui se manifeste en général 1 à 2 h après l'injection du composé. Dans tous les cas, les vomissements sont peu abondants et durent entre 4 et 5 h. Des prises de sang ont été effectuées à intervalles réguliers et les résultats sont compilés dans les tableaux IX, X et XI. Le jour $J_0$ est le jour du traitement, et un prélèvement a été effectué avant l'injection du composé. Les résultats du jour $J_0$ sont donc des valeurs contrôles. $J_1$ = premier jour après le traitement, $J_2$ = deuxième jour, etc.

Le babouin No 2 (150 mg/kg) est décédé au jour $J_{11}$, très certainement à cause de la néphrotoxicité du composé (créatine à 1552) en l'absence d'hydratation. Il faut noter que ce babouin n'a ni mangé ni bu pendant 11 d. Cette toxicité rénale est en accord avec les connaissances actuelles déjà rapportées pour des complexes du platine de ce type en l'absence d'hydratation.

Il ressort de ces essais que le PHIC à 100 mg/kg, en l'absence d'hydratation, n'a pas provoqué de néphrotoxicité et que, sous hydratation, on n'a pas observé non plus de toxicité à la dose de 200 mg/kg.

Un test d'ototoxicité a été effectué sur le babouin No 3 (200 mg/kg) au jour $J_{31}$. Ce test consiste:

a) à mesurer les potentiels évoqués auditifs lorsque l'oreille est soumise à un certain nombre (1500) de stimuli (90, 80, 70, 60, 50, 40, 30, 20 et 10 dB). Les enregistrements donnent les temps de latence de conduction de l'oreille au cerveau;

b) à réaliser le même genre d'expérience par échocochléographie. Cette analyse est beaucoup plus fine que la précédente, car elle n'est effectuée qu'au niveau de l'organe de Corti (cochlée). Les fréquences utilisées étaient de 8000, 4000, 2000 et 1000 Hz (90, 70, 50, 30, 20 et 10 dB). Les mesures ont été faites avec un appareil du type Racia Medelec Empled $MK_4$ 1026. Aucun effet ototoxique n'a pu être détecté à la dose de 200 mg/kg de PHIC. Les propriétés du PHIC comparées à celles du PDD peuvent se résumer ainsi:

| | PHIC | PDD |
|---|---|---|
| 1. Hydrosolubilité | infinie (>1500 mg/ml) | 2 mg/ml |
| 2. Propriétés antitumorales | | |
| a) Index thérapeutique | | |
| L1210 | 36,3 | 6,5 |
| S 180 | 21,5 | 5,2 |
| b) Animaux survivants avec la leucémie L1210 et le sarcome 180 | oui | non |
| 3. Néphrotoxicité à la $DL_0$ chez la souris | non | oui |

Le composé de la présente invention est supérieur au PDD selon des critères de solubilité, de toxicité et d'activité antitumorale (cf. tableaux IX, X et XI).

*Tableau IX*

Analyses du babouin No 1 : 100 mg/kg

| | 0 contrôle | $J_1$ | $J_2$ | $J_6$ | $J_{16}$ | $J_{30}$ | $J_{37}$ | $J_{38}$ | $J_{41}$ | $J_{48}$ |
|---|---|---|---|---|---|---|---|---|---|---|
| Quick Taux de prothrombine (100%) | 100 | 75 | 80 | 65 | 95 | 85 | 85 | 85 | 100 | 95 |
| Fibrinogène I (2-4 g/l) | 3,20 | — | 2,25 | 2,40 | 1,95 | 1,52 | 1,46 | 1,85 | 2,72 | 2,70 |
| Proaccélérine V (100%) | | — | — | 100 | — | — | — | — | — | — |
| Proconvertine VII + facteur Stuart X (100%) | — | — | — | 70 | — | — | — | — | — | — |
| Prothrombine II (100%) | — | — | — | 95 | — | — | — | — | — | — |
| Créatinine (53-130 µmol/1) | 117 | — | 110 | 91 | 105 | 138 | 99 | 85 | 102 | 136 |
| Granulocytes (4000-7000) | 2550 | — | — | 2480 | 4560 | 3969 | 2989 | 4824 | 4212 | 3696 |
| Plaquettes (100 000-250 000) | 364 000 | — | — | 526 000 | 414 000 | 414 000 | 362 000 | 299 000 | 371 000 | 708 000 |
| Poids (kg) | 8,4 | — | — | 7,7 | 8,4 | — | 8,35 | — | — | 7,6 |

$J_0$ = 1re injection, $J_{37}$ = 2e injection. Les valeurs entre parenthèses indiquent les normes rencontrées chez l'homme.

*Tableau IX (suite)*

Analyses du babouin No 1 : 100 mg/kg

| | $J_{57}$ | $J_{58}$ | $J_{62}$ | $J_{76}$ | $J_{79}$ | $J_{84}$ | $J_{85}$ | $J_{90}$ | $J_{100}$ |
|---|---|---|---|---|---|---|---|---|---|
| Quick Taux de prothrombine (100%) | 90 | — | 50 | 95 | 95 | — | 85 | — | 95 |
| Fibrinogène I (2-4 g/l) | 2,67 | — | 1,86 | 1,75 | 2,10 | — | 2,08 | — | 2,50 |
| Proaccélérine V (100%) | — | — | — | — | — | — | 100 | — | — |
| Proconvertine VII + facteur Stuart X (100%) | — | — | — | — | — | — | 90 | — | — |
| Prothrombine II (100%) | — | — | — | — | — | — | — | — | — |

*Tableau IX (suite)*
Analyses du babouin No 1: 100 mg/kg

| | $J_{57}$ | $J_{58}$ | $J_{62}$ | $J_{76}$ | $J_{79}$ | $J_{84}$ | $J_{85}$ | $J_{90}$ | $J_{100}$ |
|---|---|---|---|---|---|---|---|---|---|
| Créatinine (53-130 µmol/1) | 96 | — | 136 | 121 | 138 | — | 97 | 96 | 110 |
| Granulocytes (4000-7000) | 2498 | — | 3726 | 1071 | 2541 | — | 3599 | 1917 | 3230 |
| Plaquettes (100 000-250 000) | 329 000 | — | 378 000 | 375 000 | 382 000 | — | 549 000 | 685 000 | 340 000 |
| Poids (kg) | — | 8,45 | — | — | — | 7,93 | — | — | — |

$J_{58}$ = 3e injection, $J_{84}$ = 4e injection. Les valeurs entre parenthèses indiquent les normes rencontrées chez l'homme.

*Tableau IX (suite)*
Analyses du babouin No 1: 100 mg/kg

| | $J_{105}$ | $J_{106}$ | $J_{111}$ | $J_{122}$ | $J_{129}$ | $J_{133}$ | $J_{134}$ | $J_{140}$ |
|---|---|---|---|---|---|---|---|---|
| Quick Taux de prothrombine (100%) | 95 | 75 | 48 | 100 | 100 | — | — | 100 |
| Fibrinogène I (2-4 g/l) | 2,25 | 2,20 | 2,20 | 2,10 | 2,15 | — | — | 2,50 |
| Proaccélérine V (100%) | — | — | 100 | — | — | — | — | — |
| Proconvertine VII + facteur Stuart X (100%) | — | — | 39 | — | — | — | — | — |
| Prothrombine II (100%) | — | — | 70 | — | — | — | — | — |
| Créatinine (53-130 µmol/1) | 60 | 120 | 102 | * | 90 | — | 126 | 114 |
| Granulocytes (4000-7000) | 2030 | 10 120 | 1121 | 1232 | 5406 | — | 7245 | 4794 |
| Plaquettes (100 000-250 000) | 266 000 | 231 000 | 345 000 | 348 000 | 508 000 | — | 580 000 | 441 000 |
| Poids (kg) | 8,59 | — | — | — | — | 7,65 | — | — |

$J_{105}$ = 5e injection, $J_{133}$ = 6e injection. Les valeurs entre parenthèses indiquent les normes rencontrées chez l'homme.
* BUN = 4,5 mmol/l (valeurs normales comprises entre 3 et 7 mmol/l).

*Tableau X*
Analyses du babouin N° 2: 150 mg/kg

| | $J_o$ (contrôle) | $J_o$ (contrôle) | $J_1$ | $J_4$ | $J_{11}$ |
|---|---|---|---|---|---|
| Quick<br>Taux de prothrombine<br>(100%) | 95 | 90 | 60 | 85 | 55 |
| Fibrinogène I<br>(2-4 g/l) | 1,85 | 1,46 | 1,46 | 2,82 | 4,20 |
| Proaccélérine V<br>(100%) | — | — | 70 | — | 100 |
| Proconvertine VII + facteur Stuart X<br>(100%) | — | — | 62 | — | 55 |
| Prothrombine II<br>(100%) | — | — | 42 | — | 55 |
| Créatinine<br>(53-100µmol/1) | 125 | 130 | 96 | 245 | 1552 |
| Granulocytes<br>(4000-7000) | 3072 | 6885 | 9672 | 1988 | 555 |
| Plaquettes<br>(100 000-250 000) | 507 000 | 293 000 | 261 000 | 212 000 | 174 000 |
| Poids<br>(kg) | — | 7,3 | — | — | 5,5 |

$J_o$ = 1re injection. Les valeurs entre parenthèses indiquent les normes rencontrées chez l'homme.

*Tableau XI*
Analyses du babouin N° 3: 200 mg/kg

| | $J_o$ (contrôle) | $J_4$ | $J_6$ | $J_{12}$ | $J_{15}$ | $J_{18}$ | $J_{24}$ | $J_{30}$ | $J_{40}$ |
|---|---|---|---|---|---|---|---|---|---|
| Quick<br>Taux de prothrombine<br>(100%) | 95 | 10 | 30 | 85 | 90 | 100 | 100 | 95 | 95 |
| Fibrinogène I<br>(2-4 g/l) | 2,07 | 2,70 | 3,00 | 4,80 | 3,85 | 2,60 | 2,00 | 2,25 | 1,50 |
| Proaccélérine V<br>(100%) | — | 100 | 100 | — | — | — | — | — | — |
| Proconvertine VII<br>+ facteur Stuart X<br>(100%) | — | <5 | 25 | — | — | — | — | — | — |
| Prothrombine II<br>(100%) | — | 30 | 25 | — | — | — | — | — | — |
| Créatinine<br>(53-130 µmol/1) | 62 | 140 | 350 | 646 | 265 | 118 | 97 | 104 | 94 |

*Tableau XI (suite)*
Analyses du babouin N° 3: 200 mg/kg

| | $J_0$ contrôle | $J_4$ | $J_6$ | $J_{12}$ | $J_{15}$ | $J_{18}$ | $J_{24}$ | $J_{30}$ | $J_{40}$ |
|---|---|---|---|---|---|---|---|---|---|
| Granulocytes (4000-7000) | 3800 | 4536 | 5236 | 7905 | 3550 | 722 | 4872 | 10752 | 4731 |
| Plaquettes (100000-250000) | 241 000 | 70000 | 208000 | 241 000 | 114000 | 312000 | 366000 | 457000 | 299000 |
| Poids (kg) | 7,5 | 6,25 | — | 5,31 | — | — | 5,6 | 5,9 | 6,33 |

$J_0$ = 1re injection. Les valeurs entre parenthèses indiquent les normes rencontrées chez l'homme.

Le PHIC manifeste, en outre, une activité antimicrobienne et il peut être utilisé comme désinfectant.

En tant qu'agent antitumoral, le complexe de l'invention peut être administré par voie orale, intramusculaire ou intraveineuse.

Il peut être administré sous forme de capsule, poudre, comprimé ou soluté injectable, associé à des véhicules et excipients pharmaceutiquement acceptables.

Les doses moyennes administrables journellement peuvent varier de 1 à 400 mg/kg de poids corporel.

## Revendications

1. Complexe organique du platine qui est le cisisocitrato(1,2-diaminocyclohexane)platine (II).

2. Complexe organique du platine selon la revendication 1, caractérisé en ce qu'il résulte de l'interaction d'un composé du platine partiellement chélaté avec le 1,2-diaminocyclohexane, répondant à la formule générale suivante:

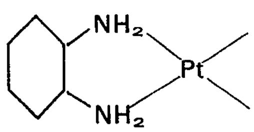

(I)

avec l'isocitrate trisodique.

3. Procédé de préparation du complexe organique du platine selon l'une des revendications 1 ou 2, caractérisé en ce qu'il consiste à faire réagir le cisdichloro(1,2-diaminocyclohexane)platine (II) avec l'isocitrate trisodique.

4. Procédé selon la revendication 3, caractérisé en ce que le cisdichloro(1,2-diaminocyclohexane)platine (II) est mis d'abord à réagir en milieu aqueux avec le nitrate d'argent, pour former le dinitrate du composé de platine correspondant que l'on fait ensuite réagir avec l'isocitrate trisodique pour obtenir le complexe de platine recherché, que l'on isole ensuite du milieu réactionnel.

5. Composition pharmaceutique possédant notamment une activité antitumorale, caractérisée en ce qu'elle comprend, à titre de principe actif, le cis-isocitrato(1,2-diaminocyclohexane)platine (II) en association avec des solvants ou des excipients pharmaceutiquement acceptables.

6. Composition pharmaceutique selon la revendication 5, caractérisée en ce qu'elle est présentée sous une forme appropriée pour administration orale, intramusculaire ou intraveineuse.

## Patentansprüche

1. Organischer Platinkomplex, der das (1,2-Diaminocyclohexan)cisisocitratoplatin (II) ist.

2. Organischer Platinkomplex gemäss Anspruch 1, dadurch gekennzeichnet, dass er aus einer Reaktion einer Platin (II) verbindung, die teilweise mit 1,2-Diaminocyclohexan chelatisiert ist und der nachfolgenden allgemeinen Formel entspricht:

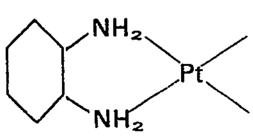

(I)

mit Trinatriumisocitrat hervorgeht.

3. Verfahren zur Herstellung des organischen Platinkomplexes gemäss einem der Ansprüche 1 oder 2, dadurch gekennzeichnet, dass es daraus besteht, dass (1,2-Diaminocyclohexan)cis-dichloroplatin (II) mit Trinatriumisocitrat zur Reaktion gebracht wird.

4. Verfahren gemäss Anspruch 3, dadurch gekennzeichnet, dass (1,2-Diaminocyclohexan)-cisdichloroplatin (II) zunächst in wässeriger Lösung zur Reaktion mit Silbernitrat gebracht wird, um das Dinitrat der entsprechenden Platinverbindung zu bilden, das man anschliessend mit dem Trinatriumisocitrat reagieren lässt, um den gesuchten Platinkomplex zu erhalten, den man dann aus dem Reaktionsmedium isoliert.

5. Pharmazeutische Zusammensetzung von insbesondere antitumoraler Wirksamkeit, dadurch gekennzeichnet, dass sie als Wirkstoff (1,2-Diaminocyclohexan)cisisocitratoplatin (II) zusammen mit pharmazeutisch annehmbaren Lösungsmitteln oder Trägern aufweist.

6. Pharmazeutische Zusammensetzung gemäss Anspruch 5, dadurch gekennzeichnet, dass sie in einer für die orale, untramusculäre oder intravenöse Verabreichung geeigneten Form dargeboten wird.

## Claims

1. Organic complex of platinum which is the cisisocitrato(1,2-diaminocyclohexane)platinum (II).

2. Organic complex of platinum according to claim 1, characterized in that it results from the interaction between a platinum compound partially chelated with 1,2-diaminocyclohexane, having the general formula:

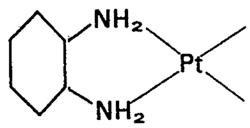

(I)

with trisodium isocitrate.

3. Process for preparing an organic complex of platinum according to one of claims 1 or 2, characterized in that cisdichloro(1,2-diaminocyclohexane)platinum (II) is reacted with trisodium isocitrate.

4. Process according to claim 3, characterized in that cisdichloro(1,2-diaminocyclohexane)-platinum (II) is first reacted in aqueous medium with silver nitrate to form the dinitrate of the corresponding platinum compound which is thereafter reacted with trisodium isocitrate to form the required platinum complex which is then recovered from the reaction mixture.

5. Pharmaceutical composition having antitumour activity, characterized in that it comprises, as active principle, the cisisocitrato(1,2-diaminocyclohexane)platinum (II) and pharmaceutically acceptable solvents or excipients.

6. Pharmaceutical composition as claimed in claim 5, characterized in that it is in a form suitable for oral, intramuscular or intravenous administration.

Fig.1

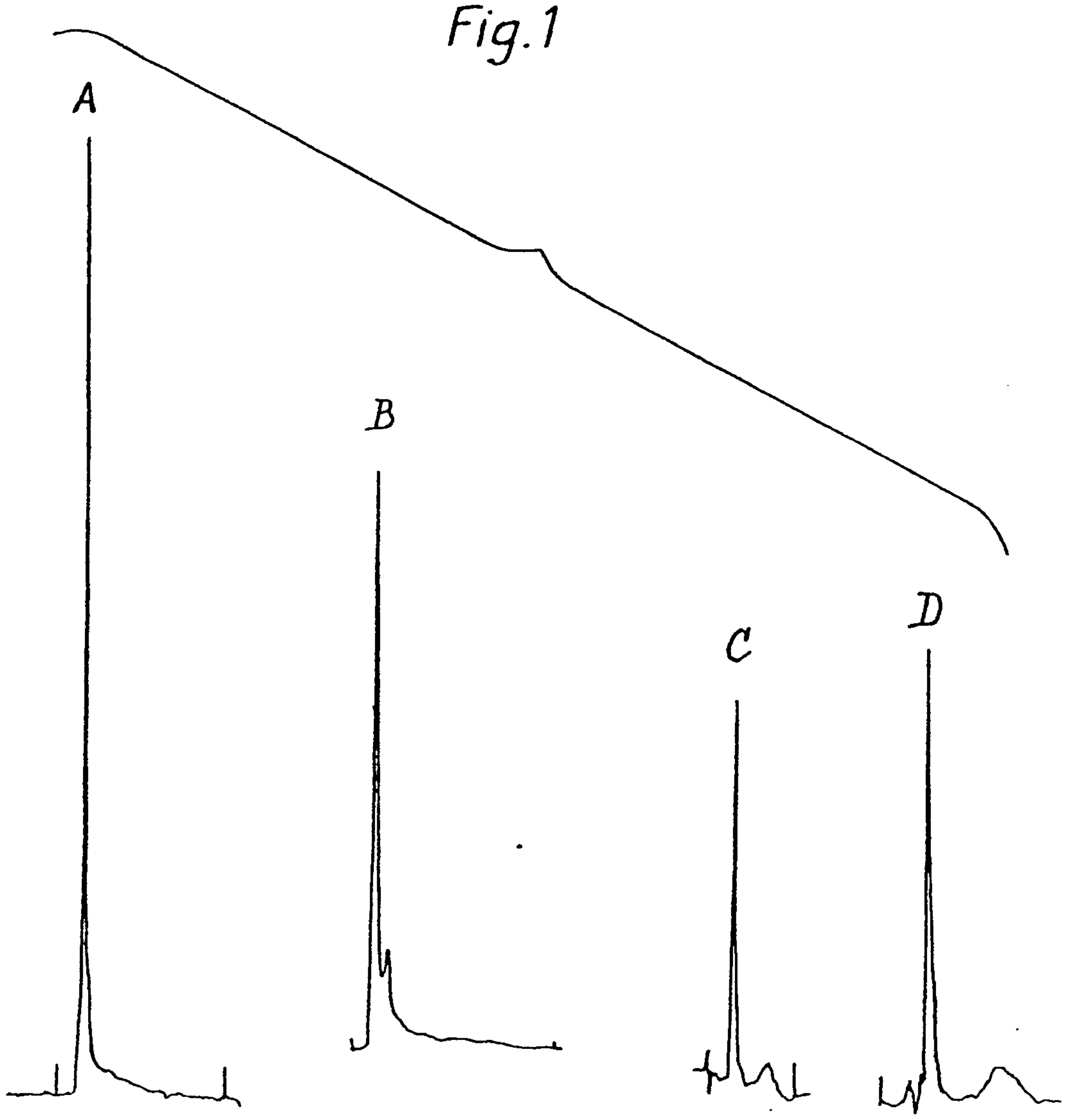

Fig. 2

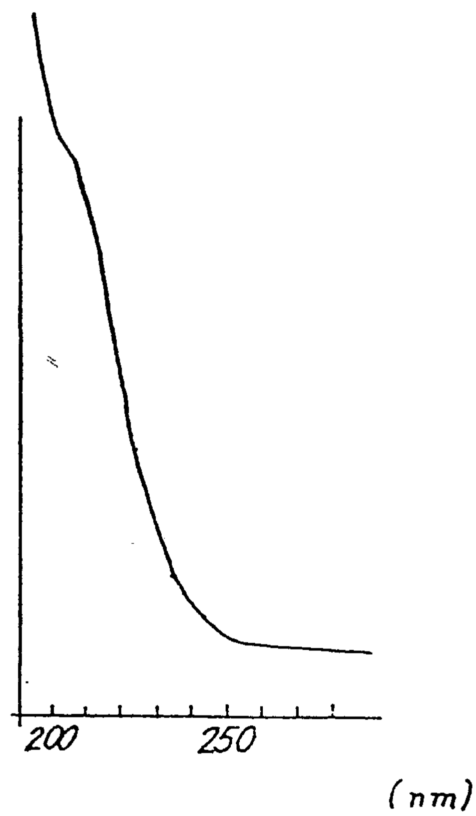


Fig. 4

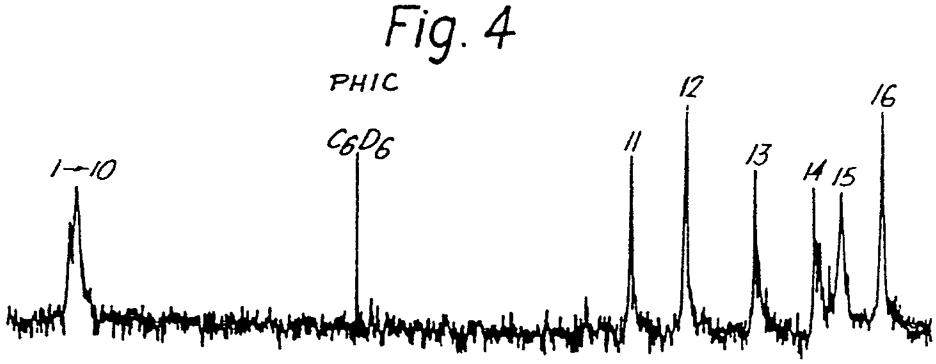

Fig.3.

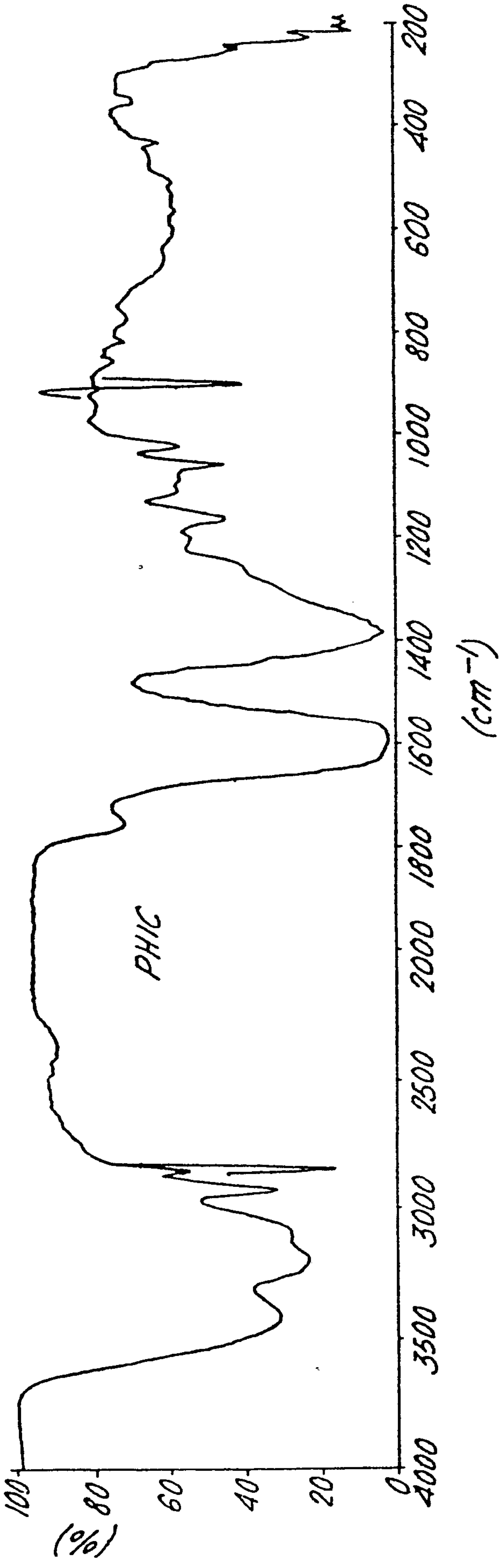
PHIC
100
80
60
40
20
0
(%)
4000
3500
3000
2500
2000
1800
1600
1400
1200
1000
800
600
400
200
(cm⁻¹)